# EUROPEAN PATENT SPECIFICATION

(11) **EP 3 219 363 B1**
(45) Date of publication and mention of the grant of the patent: **08.05.2019**
(21) Application number: 17160390.5
(22) Date of filing: 10.03.2017
(51) Int. Cl.: A61Q 1/06, A45D 40/16, A61K 8/02

(54) **A COSMETIC ELEMENT AND A METHOD FOR MAKING IT**
KOSMETISCHES ELEMENT UND VERFAHREN ZUR DESSEN HERSTELLUNG
ÉLÉMENT DE COSMÉTIQUE ET PROCÉDÉ DE SA FABRICATION

(30) Priority: 14.03.2016 IT UA20161622
(43) Date of publication of application: 20.09.2017
(73) Proprietor: Chromavis S.p.A., 20122 Milano (IT)
(72) Inventor: ROSSI, Riccardo, 27010 CERANOVA (PV) (IT); DONIDA, Giulia Maria, 26015 SORESINA (CR) (IT)
(74) Representative: Vanosi, Adelio Valeriano

(56) References cited:
- EP-A1- 0 974 332
- WO-A1-2015/140733
- US-A- 5 486 355
- US-A- 5 846 520
- US-A1- 2005 191 337
- Anonymous: "Lipstick Manufacture", , 10 May 2006 (2006-05-10), XP055321718, Retrieved from the Internet: URL:http://www.silverson.de/images/uploads /documents/TLipstick.pdf [retrieved on 2016-11-22]
- Anonymous: "Nivea Fruity Shine lip balm (SPF10)- Cherry: Review", , 7 June 2013 (2013-06-07), XP055321699, Retrieved from the Internet: URL:http://www.theindianbeauty.com/2013/06 /nivea-fruity-shine-cherry-review.html [retrieved on 2016-11-22]
- Anonymous: "Nivea Waterlily & Oil shower gel: Review", , 9 June 2013 (2013-06-09), XP055321700, Retrieved from the Internet: URL:http://www.theindianbeauty.com/2013/07 /nivea-waterlily-oil-shower-gel-review.htm l [retrieved on 2016-11-22]

## Description

### FIELD OF THE INVENTION

The present invention relates to a cosmetic element and to a manufacturing method thereof.

In particular, it relates to a cosmetic element for make-up, such as a product for lips, eyes, face, body, such as foundation, eye shadow, blush, bronzer or lip balm.

### PRIOR ART

Some make-up products, such as lipsticks or lip balms, are marketed in the form of elongated sticks contained in conventional machines or the like or more recently, in the form of a cylinder of diameter and height of 2-4 cm with hemispherical surface shape, attached to a container fitted with a closure.

The containers can assume even very original shapes, such as spheres or cylinders with transparent dome closure that 'reproduces' the profile of the lip balm contained therein.

Originality in the design of container does not goes hand in hand with the aesthetic appearance of the lip balm contained therein that, apart from a few examples in which tenuous colorants are added to a usually anhydrous matrix which constitutes the balm, are little captivating and aesthetically very simple and dull.

### SUMMARY OF THE INVENTION

An object of the present invention is to provide a cosmetic element, in particular a make-up element, with an attractive and original design, valuable both before a first use or after it.

This and other objects are achieved by making a cosmetic element according to the technical teachings of the appended claims.

An advantage of the invention is to make a cosmetic element that helps to effectively preserve any colorant products or active ingredients included in the cosmetic element itself, until ready to use.

### BRIEF DESCRIPTION OF THE FIGURES

Further features and advantages of the invention will become apparent from the description of a preferred but non-exclusive embodiment of the invention, shown by way of a non-limiting example in the accompanying drawings, in which:
figures 1 to 3 are simplified sectional views of different manufacturing steps of the cosmetic element according to the present invention;
figure 1A is a plan view of a part used in the method of the previous figures;
figure 4 schematically shows a sectional view of a final step of the method shown in figures 1-3;
figure 5 shows a lateral view of a cosmetic designed according to the previous steps and, in section, a cover to be applied to the cosmetic;
figures 6 and 7 show in simplified section, some steps of an alternative method of manufacture of the cosmetic element according to the present invention;
figures 8 and 9 show in simplified section, some steps of a further method of manufacture of the cosmetic element according to the present invention;
figure 10 shows a lateral view of a cosmetic designed according to the steps shown in figures 8 and 9;
figures 11 to 14 show in simplified section, some steps of yet a different method of manufacture of the cosmetic element according to the present invention;
figure 15 shows a lateral view of a cosmetic designed according to the steps shown in figures 11-14; and
figure 16 is an enlargement of the portion enclosed by a circle in figure 6.

### DETAILED DESCRIPTION OF THE INVENTION

With reference to the above figures, a cosmetic is shown, globally denoted by reference numeral 1.

In particular, the cosmetic is a make-up product for lips, eyes, face, body, such as foundation, eye shadow, blush, bronzer, gloss, concealer or lip balm.

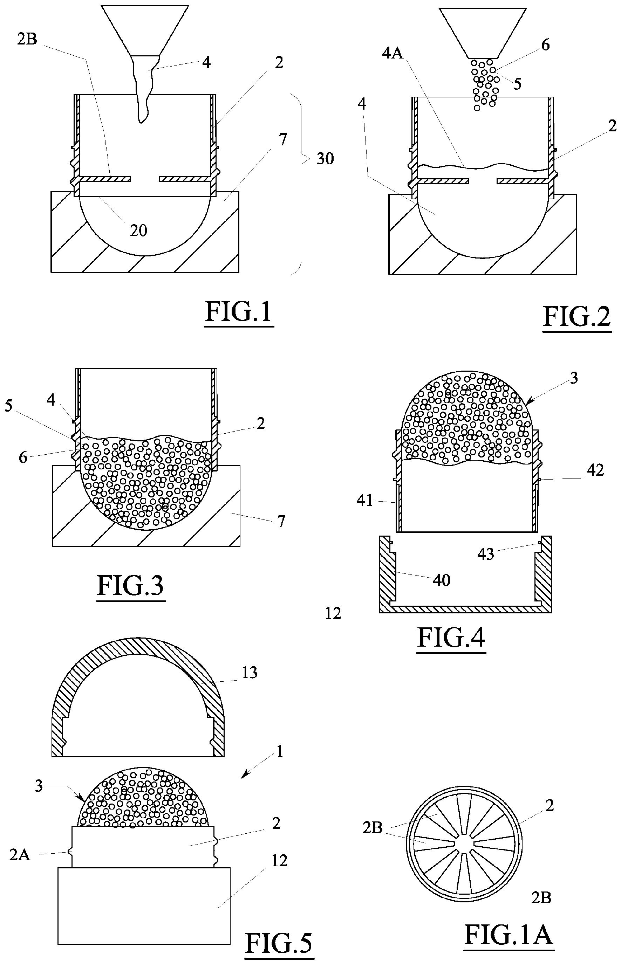

In particular, the cosmetic 1 shown in figure 5 comprises a containing structure 2 of the cosmetic element 3 which can be attached, for example by pressure, to a base 12. The containing structure 2 may have a thread 2A for coupling with a closure 13, advantageously transparent and dome shaped. Of course, the representation in figure 5 is purely illustrative and the cosmetic element may be accommodated in containers of different shape or configuration, some of which will also be described hereinafter.

As mentioned above, figures 1 to 4 show subsequent manufacturing steps of the cosmetic element 3.

First, a containing structure 2 (known in cosmetics by the name of grid) is associated, at an opening 20 thereof, to a first matrix 7.

Matrix 7 is preferably totally made of silicon, of the type used in cosmetics, such as for making lipsticks. It may however be made also of other materials including metal, resin, PP, etc.

In the present description, matrix 7 has a cavity with a shape of a hemisphere, but it may have different shapes. The cavity of the matrix 7 is configured to define the final shape of at least a visible part of the final cosmetic product.

Basically, as shown in figure 1, the coupled containing structure 2 and matrix 7 form a mould in which, in a first step, a first cosmetic product 4 is poured.

The first cosmetic product 4 may be a matrix (or texture) with preferably anhydrous base, which may comprise waxes, oils and/or solvents and rheological additives, and optionally colorants and volatile solvents.

A possible formulation of the first cosmetic product 4 is as follows: 80-90% emollient oils, 10-20% rheological additives, 0.1-1% antioxidants.

An alternative formulation of the first cosmetic product 4, such as for use as a lipstick, is as follows: 50-90% emollient oils, 30-50% waxes, 10-20% rheological additives, 0.1-1% antioxidants, 0-30% colorants and/or beads.

According to the present invention, prior to pouring, the first cosmetic product 4 is heated at a temperature higher than the melting temperature, so that it takes a liquid texture.

In the example, the temperature at which the first cosmetic product is heated is of between 60° and 130°, preferably between 75° and 110°.

As is seen in figure 2, a quantity of first cosmetic product 4 is poured in mould 30 which may be such that the free surface 4A of the first product submerges fins 2B radially protruding from the inner surface of the containing structure 2.

In figure 1A, the mould is shown from above and the arrangement of fins 2B is clearly visible. Of course, in the present text 2, the shape of the containing structure is purely exemplary and in different variants, containing structures with different shape may be used, advantageously provided with portions similar to the fins intended to be 'sunk' in the cosmetic element 3 once solidified.

It should be noted that in this configuration, the interface surface between the containing structure 2 and matrix 7 does not need any sealing means, the latter being made of silicone. If necessary, suitable sealing means may of course be provided between the containing structure 2 and matrix 7, adapted to prevent or limit the leakage of the first liquid cosmetic product 4 outside the cavity of mould 30.

Once the first cosmetic product 4 has been poured, it is possible to wait for a time usually of between 3 and 60 seconds to allow the solidification of the first cosmetic product 4 only at an interface surface with mould 30, and in particular with matrix 7.

Thereafter, while the first cosmetic product 4 inside mould 30 is still at least partially liquid, a plurality of macroscopic corpuscular units 6, formed by at least a second cosmetic product 5, is included inside the first cosmetic product.

This operation may be carried out as shown in figure 2, by spreading a plurality of preformed macroscopic corpuscular units 6 above the free surface 4A of the first cosmetic product 4, so that they sink inside the first cosmetic product 4 before said first cosmetic product 4, as it cools down, has a completely solid texture.

Advantageously, as already mentioned, the macroscopic corpuscular units 6 are poured or spread on the free surface 4A of the first cosmetic product when the latter has already partially solidified on the surface of matrix 7.

In this way, the macroscopic corpuscular units 6 sink up to settle on a solidified layer of first cosmetic product provided at the interface with matrix 7, and thus they do not appear directly on the application surface of the cosmetic element 3. The latter can in fact be defined only by the first cosmetic product 4, so as to isolate the macroscopic corpuscular units 6 from the external environment until a first use of the cosmetic element itself.

As already mentioned, the macroscopic corpuscular units 6 are formed in at least a second cosmetics product 5 different from the first cosmetic product 4. The two cosmetic products may differ in substantive features or for example even just in the colour, thus having a very similar formulation.

In the example described, each macroscopic corpuscular unit 6 may be made as an aggregate of cosmetic powder and binder, obtained for example by processing in coating pan, whether or not followed by a drying step.

They can have any desired shape and can be produced with any methodology suitable for the purpose. Purely by way of example, they can take the shape of beads, pellets, solids with random edges, hearts, stars, triangles, squares, rectangles, strips, cubes etc.

Advantageously, the macroscopic corpuscular units 6 are clearly visible to the naked eye and have a maximum diameter of between 2 and 8 mm, preferably between 4 and 6 mm.

In the example, the second cosmetic product 5 therefore comprises: powders, binders, oils, volatile solvents, texturizers, dyes and/or beads, filming agents, rheological additives, active ingredients such as vitamins, moisturizers, emollients, plant extracts preferably selected so as to not have a physical phase transition at the pouring temperature of the first cosmetic product 4.

Of course, macroscopic corpuscular units 6 may be used for the production of the cosmetic element 3 that are made in any other formulation or with any other methodology.

Once the macroscopic corpuscular units 6 are sunk in the first cosmetic product 4 up to contact, as mentioned above, with the possible already solidified layer of the latter at the interface surface with matrix 7, the configuration shown in figure 3 is obtained.

Optionally, it is possible to further pour the first cosmetic product 4 until the level of the first cosmetic product reaches the desired height.

A first cooling of the cosmetic element 3 and of mould 30 is then carried out, for example at room temperature for between 3 and 10 minutes after which matrix 7 is separated from the cosmetic element 3.

It should be said that this first cooling has the sole purpose of allowing the separation of matrix 7 from the cosmetic element 3 and of allowing the safe handling of the cosmetic element itself.

Thereafter, the cosmetic element 3, already constrained to the containing structure 2, can be associated snap-wise by means of the latter to base 12.

In order to prevent the reciprocal rotation between the base and the containing structure, torsional coupling means may be provided therebetween, which may for example comprise protuberances 40 made on the base which engage into corresponding grooves 41 of the containing structure. Advantageously, the mutual constraint is by undercut coupling of an annular tooth 42 provided on the containing structure with a corresponding annular tooth 43 of base 12.

After coupling with base 12, a further cooling is carried out to complete the solidification of the first cosmetic product 4. The second cooling advantageously takes place at room temperature.

In fact, the first cosmetic product 4 is selected to be solid at room temperature, with a texture useful to allow an optimal application of the make-up product.

The second cooling step can take place either before or after the application of the base and/or cover 13.

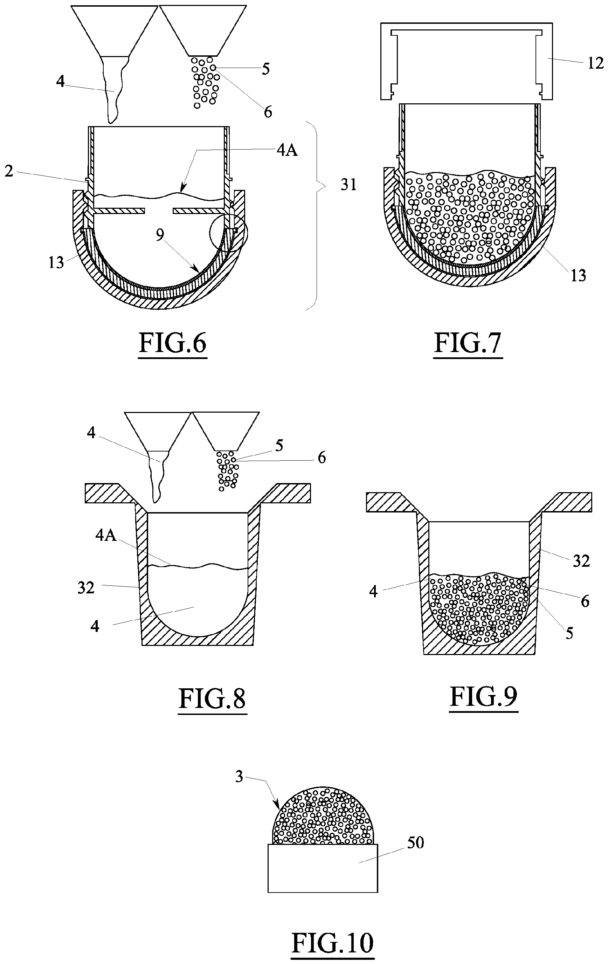

Figures 6 and 7 show some alternative manufacturing steps of the cosmetic element 3 according to the present invention.

In these figures and in the following ones, the same numeral references already cited are used to indicate corresponding parts or functionally similar parts to those already described. The description of those parts will therefore not be repeated.

The main difference with the embodiment shown above is in the use of a lens 9 coupled to closure 13 for the direct forming of the cosmetic element 3 in the container used for distributing the final cosmetic element 3.

In this case the shape of the cavity of the lens defines a final outer shape of at least a part of the cosmetic product.

Basically, in this case, mould 31 consists of lens 9 coupled to the containing structure 2. Lens 9 is held in place by closure 13 screwed right to the containing structure.

The simple analysis of the figures is enough to fully understand the manufacturing steps according to this embodiment, which in fact are virtually identical to those described above.

Also in this case, the coupling with base 12 may take place before or after a first cooling step of the cosmetic element (FIG. 7).

The structure of lens 9, which advantageously may have an undercut coupling 44 with closure 13, may provide a layer 9A of a material different from the main one 9B of which the lens is made, which promotes easy detachment between the lens itself and the cosmetic element 3 at the time of use.

The user, for example at a first use, can simply unscrew closure 13, thus removing also lens 9. Alternatively, lens 9 may be removed before entering the product on the market, in an additional step to be preferably carried out after the complete solidification of the first cosmetic product 4.

The embodiment shown herein allows minimising the production costs of the cosmetic element 3 which, immediately after pouring and cooling, is ready to be marketed with obvious saving in cost and production times, without using additional matrices.

In the embodiment shown in figures 8 and 9, a mould 32 made in one piece is used. The filling and manufacturing steps of the cosmetic element 3 are totally similar to those described above.

Once the extraction of the cosmetic element 3 from the mould has been carried out, it is constrained by interference to a support 50 (fig. 10), which may be of conventional type as that used for lipsticks or other type. Of course, the support may be inserted in any type of container.

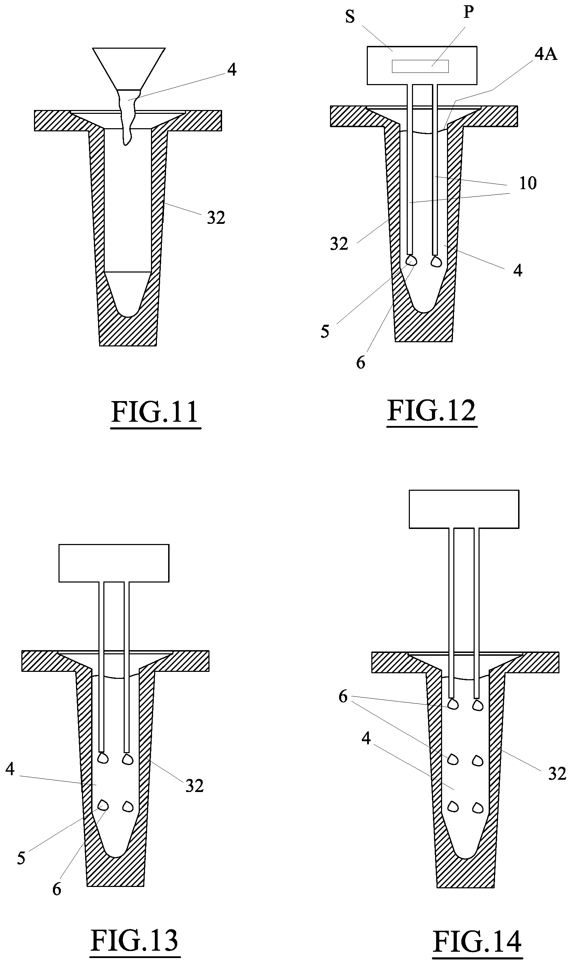

Figures 11 to 14 show a different embodiment of the method according to the invention.

In this case, an elongated silicone mould 32 is used, very similar to the one used for forming lipsticks.

Also in this case a cavity of the silicone mould 32 defines the final outer shape of at least a part of the cosmetic product.

The first step is identical to those described above and provides for pouring the first cosmetic product 4 in the cavity of mould 32.

Thereafter, at least one needle 10 is dropped (immersed) in the cosmetic product 4 not yet solidified (two are dropped in the example but as many needles may be provided as needed).

Each needle 10 is fed by a positive-displacement pump P connected to a tank S, preferably at a controlled temperature.

The second cosmetic product contained in tank S is injected through needles 10 in a predefined volumetric amount so as to form a macroscopic corpuscular unit 6 in the first cosmetic product 4, for each needle 10 as shown in figure 12.

The needle can remain in the same position for the entire duration of the injection, as shown in the drawings, or be moved during the injection to 'draw' shapes inside the first cosmetic product, such as strips, circles, hearts, stars, etc.

After the first injection, needle 10 may be moved inside the first cosmetic product 4 still in a liquid state (fig. 13) and the injection operation may be repeated. The needle may be moved again (fig. 14) and a further amount of second cosmetic product 5 may be injected.

Of course, the operation can be repeated as many times as necessary to obtain a cosmetic element 3 with the desired configuration.

In this embodiment, as in the other, the second cosmetic product 5 can have a formulation very similar to the first cosmetic product 4 and can be injected (possibly after being brought to the melting temperature) inside tank S.

Once the injection steps have ended, the cosmetic element 3 may be cooled, advantageously within refrigeration units already present in conventional machines for lipsticks.

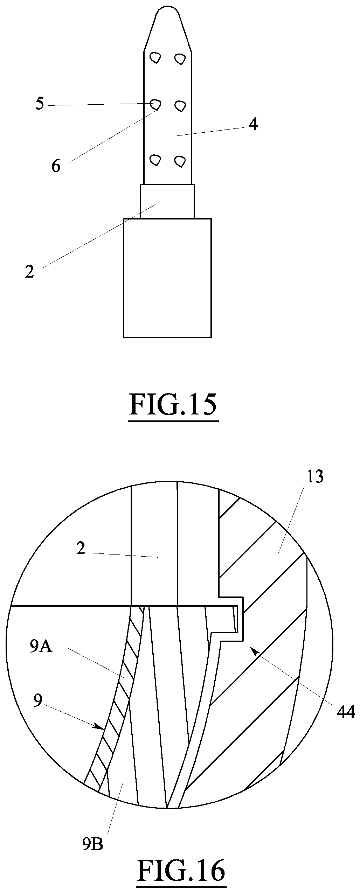

After cooling, the stick may be removed from the mould and coupled with a machine for lipsticks like that shown in figure 15, or with any other conventional container.

In the embodiments described above, referenced has been made to a first cosmetic product 4 and at least a second cosmetics product 5, which are different from each other in at least one property which may also be only the colour.

Of course, however, the first 4 and the second 5 cosmetic product may be different also in other aspects, from the formulation to the very nature of the product.

The cosmetic element 3 produced through the described methodologies is particularly advantageous because, before a first use, the macroscopic corpuscular units 6 may be protected from direct contact with the external environment by a more or less thin layer of first cosmetic product 4, which protects and isolates them, also hiding them to the eye if the first cosmetic product is opaque or non-transparent or coloured.

The 'insulating' layer of the first cosmetic product is created naturally as a result of the cooling of the part of first cosmetic product 4 that comes into contact with the surface of the mould, in particular with matrix 7 or lens 9.

After the first use of the cosmetic element 3, the outer layer of first cosmetic product 4 is removed by the user, thus exposing the macroscopic corpuscular units 6. This provides an appealing aesthetic effect and ensures the integrity of any active ingredients present in the second cosmetic product 5 of which the macroscopic corpuscular units 6 are made.

In a preferred embodiment, the first cosmetic product 4, at least when is solidified, is transparent to light, thus allowing a user to see the plurality of macroscopic corpuscular units 6 included in the matrix consisting of the first cosmetic product 4. This creates a very pleasant aesthetic effect that makes the product made according to the present invention unique and captivating.

It is noted that all the percentages given in the present description are to be understood by weight. The other measurements are instead carried out at 25 degrees centigrade.

Various embodiments of the invention have been described but others may be conceived using the same innovative concept.

## Claims

1. Cosmetic element (3), in particular a make-up element, formed by a first cosmetic product (4) and at least a second cosmetic product (5) different from the first one, the first cosmetic product (4) being a matrix inside of which a plurality of macroscopic corpuscular units (6) are sunk, the macroscopic corpuscular units (6) being formed by said at least one second cosmetic product (5), **characterized in that** a whole surface of application of the cosmetic element, in a first use configuration, is defined only by said first cosmetic product (4).

2. Cosmetic element (3) according to one or more of the previous claims, wherein the first cosmetic product (4) is transparent or semi-transparent, so as to make the macroscopic corpuscular units (6) visible through the first cosmetic product.

3. Cosmetic (1) comprising a containing structure (2) constrained to said cosmetic product (3), and/or wherein the containing structure (2) comprises at least a portion (2B) thereof buried in said cosmetic element (3).

4. Method for making a cosmetic element (3) comprising the steps of:
a. heating a first cosmetic product (4) at a temperature higher than the melting temperature, so that it has a liquid texture,
b. pouring the first liquid cosmetic product (4) inside a mould (30, 31) that at least partially defines the final shape of the cosmetic element (3) and,
c. after a solidification of the first cosmetic product (4) only at an interface surface with mould 30, while the first cosmetic product (4) inside the mould (30, 31) is still at least partially liquid, including a plurality of macroscopic corpuscular units (6), formed by said at least one second cosmetic product (5), inside the first cosmetic product,
d. carrying out a first cooling of the cosmetic element (3) thus obtained.

5. Method according to the previous claim, comprising the additional step of at least partially extracting the cosmetic element (3) from said mould (30, 31, 32) and, after the first cooling, further cooling the cosmetic element (3) until at least the whole cosmetic product (4) has a solid texture.

6. Method according to claim 4, wherein the inclusion of the plurality of macroscopic corpuscular units (6) in the first cosmetic product is carried out by spreading said plurality of preformed macroscopic corpuscular units (6) above the free surface of the first cosmetic product (4), so that they sink inside the first cosmetic product (4) before said first cosmetic product, as it cools down, has a completely solid texture.

7. Method according to claim 4, wherein the inclusion of the plurality of macroscopic corpuscular units (6) in the first cosmetic product (4) comprises the steps of:
a. plunging at least a needle (10) inside said first cosmetic product (4) while it is still liquid,
b. injecting a predefined amount of said second cosmetic product (5) in the liquid state through said needle (10), so as to form at least a macroscopic corpuscular unit directly inside said first liquid cosmetic product (4), and optionally, stopping the injection,
c. optionally, moving said at least one needle (10) inside the first cosmetic product (4) and
d. optionally, injecting again a predefined amount of said second cosmetic product in the liquid state through said needle (10) in the first cosmetic product (4), thus forming a further macroscopic corpuscular unit (6)
e. extracting said at least one needle from the first cosmetic product (4) while at least one part of the latter is still liquid.

8. Method according to one or more of the previous claims wherein said mould (32) is made of a single deformable elastic material, and/or wherein said mould (30) comprises a containing structure (2) closed at an end by a hollow matrix (7), and/or wherein said mould (31) comprises a containing structure (2) closed at an end by a lens (9) intended to be removed from a surface of application of the cosmetic element (3) only during a first use.

9. Cosmetic element or method according to one or more of the previous claims wherein said macroscopic corpuscular units have a maximum diameter ranging from 2 to 8 mm, preferably from 4 to 6 mm.

## Patentansprüche

1. Kosmetisches Element (3), insbesondere ein Make-up-Element, geformt aus einem ersten kosmetischen Produkt (4) und mindestens einem zweiten kosmetischen Produkt (5), das sich vom ersten unterscheidet, wobei das erste kosmetische Produkt (4) eine Matrix ist, in die eine Vielzahl makroskopischer korpuskularer Einheiten (6) versunken ist, wobei die makroskopischen korpuskularen Einheiten (6) aus dem mindestens einen zweiten kosmetischen Produkt (5) geformt sind, **dadurch gekennzeichnet, dass** eine ganze Oberfläche für die Anwendung des kosmetischen Produkts, in einer ersten Nutzungskonfiguration, nur durch das erste kosmetische Produkt (4) definiert ist.

2. Kosmetisches Element (3) nach einem oder mehreren der vorstehenden Ansprüche, wobei das erste kosmetische Produkt (4) transparent oder halbtransparent ist, so dass die makroskopischen korpuskularen Einheiten (6) durch das erste kosmetische Produkt sichtbar sind.

3. Kosmetik (1), umfassend eine Behälterstruktur (2), die auf das kosmetische Produkt (3) beschränkt ist, und/oder wobei die Behälterstruktur (2) mindestens einen Abschnitt (2B) davon enthält, der im kosmetischen Element (3) vergraben ist.

4. Verfahren zum Herstellen eines kosmetischen Elements (3), umfassend die folgenden Schritte:
a. Erwärmen eines ersten kosmetischen Produkts (4) auf eine Temperatur, die höher ist als die Schmelztemperatur, so dass es eine flüssige Textur aufweist,
b. Gießen des ersten flüssigen kosmetischen Produkts (4) in eine Form (30, 31) die die Endform des kosmetischen Elements (3) mindestens teilweise definiert, und
c. nach Erhärten des ersten kosmetischen Produkts (4) nur an einer Schnittstellenfläche zu Form 30, während das erste kosmetische Produkt (4) in der Form (30, 31) immer noch mindestens teilweise flüssig ist, Aufnehmen einer Vielzahl makroskopischer korpuskularer Einheiten (6), die von dem mindestens einen zweiten kosmetischen Produkt (5) geformt werden, in das erste kosmetische Produkt,
d. Ausführen eines ersten Kühlens des so erhaltenen kosmetischen Elements (3).

5. Verfahren nach dem vorstehenden Anspruch, umfassend den zusätzlichen Schritt des mindestens teilweisen Extrahierens des kosmetischen Elements (3) aus der Form (30, 31, 32) und, nach dem ersten Kühlen, ferner Kühlen des kosmetischen Elements (3), bis mindestens das ganze kosmetische Produkt (4) eine feste Textur aufweist.

6. Verfahren nach Anspruch 4, wobei die Einführung der Vielzahl makroskopischer korpuskularer Einheiten (6) in das erste kosmetische Produkt durch Verteilen der Vielzahl vorgeformter makroskopischer korpuskularer Einheiten (6) oberhalb der freien Oberfläche des ersten kosmetischen Produkts (4) ausgeführt wird, so dass sie in das erste kosmetische Produkt (4) einsinken, bevor das erste kosmetische Produkt, mit dem Abkühlen, eine vollständig feste Textur aufweist.

7. Verfahren nach Anspruch 4, wobei die Einführung der Vielzahl makroskopischer korpuskularer Einheiten (6) in das erste kosmetische Produkt (4) die folgenden Schritte umfasst:
a. Eintauchen mindestens einer Nadel (10) in das erste kosmetische Produkt (4), während es noch flüssig ist,
b. Injizieren einer vorgegebenen Menge des zweiten kosmetischen Produkts (5) im flüssigen Zustand durch die Nadel (10), um mindestens eine makroskopische korpuskulare Einheit direkt im ersten flüssigen kosmetischen Produkt (4) zu formen, und optional Anhalten der Injektion,
c. optional Bewegen der mindestens einen Nadel (10) im ersten kosmetischen Produkt (4) und
d. optional erneutes Injizieren einer vorgegebenen Menge des zweiten kosmetischen Produkts im flüssigen Zustand durch die Nadel (10) in das erste kosmetische Produkt (4), somit Formen einer weiteren makroskopischen korpuskularen Einheit (6)
e. Extrahieren der mindestens einen Nadel aus dem ersten kosmetischen Produkt (4), während mindestens ein Teil des letzteren noch flüssig ist.

8. Verfahren nach einem oder mehreren der vorstehenden Ansprüche, wobei die Form (32) aus einem einzigen verformbaren elastischen Material hergestellt ist, und/oder wobei die Form (30) eine Behälterstruktur (2) umfasst, die an einem Ende durch eine hohle Matrix (7) geschlossen ist, und/oder wobei die Form (31) eine Behälterstruktur (2) umfasst, die an einem Ende durch eine Linse (9) geschlossen ist, die dazu bestimmt ist, nur während einer ersten Verwendung von einer Oberfläche zur Anwendung des kosmetischen Produkts (3) entfernt zu werden.

9. Kosmetisches Element oder Verfahren nach einem oder mehreren der vorstehenden Ansprüche, wobei die makroskopischen korpuskularen Einheiten einen maximalen Durchmesser im Bereich von 2 bis 8 mm, vorzugsweise von 4 bis 6 mm aufweisen.

## Revendications

1. Élément cosmétique (3), en particulier un élément de maquillage, formé par un premier produit cosmétique (4) et au moins un deuxième produit cosmétique (5) différent du premier, le premier produit cosmétique (4) étant une matrice à l'intérieur de laquelle sont coulées une pluralité d'unités corpusculaires macroscopiques (6), les unités corpusculaires macroscopiques (6) étant formées par ledit au moins un deuxième produit cosmétique (5), **caractérisé en ce que** une surface intégrale d'application de l'élément cosmétique, dans une première configuration, est définie seulement par ledit premier produit cosmétique (4).

2. Élément cosmétique (3) selon une ou plusieurs des revendications précédentes, le premier produit cosmétique (4) étant transparent ou semi-transparent, de façon que les unités corpusculaires macroscopiques (6) soient visibles à travers le premier produit cosmétique.

3. Cosmétique (1) comprenant une structure de confinement (2) contrainte sur ledit produit cosmétique (3), et/ou la structure de confinement (2) comprenant au moins une partie (2B) de celle-ci enfouie dans ledit élément cosmétique (3).

4. Méthode de réalisation d'un élément cosmétique (3) comprenant les étapes suivantes :
a. chauffage d'un premier produit cosmétique (4) à une température supérieure à la température de fusion, afin qu'il présente une structure liquide,
b. versement du premier produit cosmétique (4) liquide dans un moule (30, 31) définissant au moins en partie la forme finale de l'élément cosmétique (3), et
c. après une solidification du premier produit cosmétique (4) seulement sur une surface d'interface avec le moule 30, le premier produit cosmétique (4) à l'intérieur du moule (30, 31) étant encore au moins partiellement liquide, incorporation d'une pluralité d'unités corpusculaires macroscopiques (6), formées par ledit au moins un deuxième produit cosmétique (5), à l'intérieur du premier produit cosmétique,
d. exécution d'un premier refroidissement de l'élément cosmétique (3) ainsi obtenu.

5. Méthode selon la revendication précédente, comprenant l'étape additionnelle de l'extraction, au moins partiellement, de l'élément cosmétique (3) dudit moule (30, 31, 32), et, à la suite du premier refroidissement, le refroidissement supplémentaire de l'élément cosmétique (3) jusqu'à ce qu'au moins l'intégralité du produit cosmétique (4) ait une structure solide.

6. Méthode selon la revendication 4, l'introduction de la pluralité d'unités corpusculaires macroscopiques (6) dans le premier produit cosmétique s'effectuant en étalant ladite pluralité d'unités corpusculaires macroscopiques (6) au-dessus de la surface libre du premier produit cosmétique (4), afin qu'ils coulent à l'intérieur du premier produit cosmétique (4) avant que ledit premier produit cosmétique n'acquière une texture entièrement solide au fur et à mesure de son refroidissement.

7. Méthode selon la revendication 4, l'introduction de la pluralité d'unités corpusculaires macroscopiques (6) dans le premier produit cosmétique (4) comprenant les étapes suivantes :
a. enfoncement d'une aiguille (10) à l'intérieur dudit premier produit cosmétique (4) alors qu'il est encore liquide,
b. injection d'une quantité prédéfinie dudit deuxième produit cosmétique (5) à l'état liquide par ladite aiguille (10) de façon à former au moins une unité corpusculaire macroscopique directement à l'intérieur dudit produit cosmétique liquide (4), et, en option, arrêt de l'injection,
c. en option, déplacement de ladite au moins une aiguille (10) à l'intérieur du premier produit cosmétique (4), et,
d. en option, injection une nouvelle fois d'une quantité prédéfinie dudit deuxième produit cosmétique à l'état liquide par ladite aiguille (10) dans le premier produit cosmétique (4), en formant ainsi une nouvelle unité corpusculaire macroscopique (6),
e. extraction de ladite au moins une aiguille hors du premier produit cosmétique (4), au moins une partie de ce dernier étant encore liquide.

8. Méthode selon une quelconque des revendications précédentes, ledit moule (32) étant composé d'une matière élastique déformable unique, et/ou ledit moule (30) comprenant une structure de confinement (2) fermée à un bout par une matrice creuse (7), et/ou ledit moule (31) comprenant une structure de confinement (2) fermée à un bout par une lentille (9) dont l'intention est de l'enlever d'une surface d'application de l'élément cosmétique (3) seulement lors d'une première utilisation.

9. Élément cosmétique ou méthode selon une ou plusieurs des revendications précédentes, le diamètre maximum desdites unités corpusculaires macroscopiques mesurant de 2 à 8 mm, de préférence de 4 à 6 mm.
